# EUROPEAN PATENT SPECIFICATION

(11) **EP 0 840 868 B1**
(45) Date of publication and mention of the grant of the patent: **16.08.2000**
(21) Application number: 96905946.8
(22) Date of filing: 11.03.1996
(51) Int. Cl.: F16L 53/00, F16L 58/10, F16L 59/14

(54) **PIPE COATING AND PIPE WITH THIS COATING**
ROHRBESCHICHTUNG UND ROHR MIT DIESER BESCHICHTUNG
REVETEMENT DE TUYAUX ET TUYAU AVEC CE REVETEMENT

(30) Priority: 10.03.1995 GB 9504863
(43) Date of publication of application: 13.05.1998
(73) Proprietor: Bredero Price Coaters Limited, London EC4V 6JA (GB); Hyperlast Limited, Birch Vale, Stockport, Cheshire SK12 5BR (GB)
(72) Inventor: MULLEN, Douglas, Thomas, Port Seton, East Lothian EH32 0NF (GB); WRIGHT, Anthony, Stockport, Cheshire SK12 5BR (GB)
(74) Representative: Ede, Eric
(86) International application number: GB9600559
(87) International publication number: WO9628684

(56) References cited:
- EP-A- 0 112 706
- EP-A- 0 132 219
- WO-A-90/05266
- GB-A- 1 181 672
- GB-A- 2 197 705

## Description

This invention is concerned with coating of pipes of the type used in subterranean or submerged pipelines for recovery of oil, gas, slurries or the like pipeline materials from a subterranean well. In particular it is concerned with pipes in which a steel pipe section is coated with an anti-corrosion coating and then an anti-buoyancy coating.

Underwater pipelines are most frequently used for transporting hydrocarbons from the off-shore reservoirs to shore. With such substances, it is important that the temperature within the pipeline does not fall below a certain level, otherwise hydrate and wax formation and deposition occur. The temperature of the hydrocarbons increases with the depth of the reservoirs. In the transportation of such materials it is necessary to prevent heat loss from the hydrocarbons and this is generally addressed by providing an insulating coating around the pipeline.

Underwater pipelines may also require a weight coating for several purposes including the need to weigh the pipeline down and so maintain the position of the pipe on the seabed and to form a protective cover around the pipeline.

Concrete coatings have in the past served this purpose. However, concrete has poor insulating properties and is therefore not ideal for this application. Furthermore, set concrete is inflexible and therefore prone to cracks or damage both during the pipe-laying process and afterwards when the pipeline is in position on the sea bed. Such damage can lead to the concrete coating falling off the pipeline and loss of the insulation for the pipeline.

British Patent No. 1 573 814 discloses a coating for a pipeline comprising a weight coating of an elastic material, for example rubber containing iron powder and ore if desired. Rubber is an expensive material and in addition has poor insulating properties.

West German Patent No. 2 544 194 discloses a flexible pipeline where an inner pipe of synthetic rubber is coated with several layers of rubber, having steel bars incorporated therein. This coating does not insulate the pipe, the function of the synthetic resin being merely to pack the open structure of the steel bars.

Considering the objective of insulation, it has been proposed to use polyurethanes as a bilayer syntactic urethane system and a typical weight coating of concrete is applied thereafter as required.

Another known type of insulation comprises layers of rubber and PVC-foam. Preformed blocks of PVC or a PVC bandage are placed around the pipe and then rubber bandages are wrapped around the blocks, so securing the PVC. The rubber coating must then be vulcanised before the pipe can be used. This method gives pipes of good quality, but they are expensive to produce. The pipes also have reduced application because of the thermal resistance of the PVC-foam (about 50-80°C).

The present invention aims to overcome the above problems associated with known pipe coatings and to provide an improved pipe coating which displays both good insulating properties and gives weight to the pipeline, which can be reeled from a dynamically positioned vehicle and which is, at the same time, easy to apply, flexible, and easy to work with.

According to the present invention there is provided a pipe having an anti-corrosion coating typically having a film thickness of from about 350 µm to about 500 µm, and a protective weight coating of a urethane based copolymer applied over said anti-corrosion coating, said protective weight coating incorporating a high density filler material.

Preferably, the high density filler material is an inorganic particulate material such as micro fine barium sulphate.

Advantageously, a slot is provided in the surface of the protective weight coating for laying an induction cable, the slot preferably being frustoconical in section, the width of the slot increasing away from the surface of the protective weight coating.

Preferably, three slots are provided in the surface of the protective weight coating, the slots being equispaced around the circumference of the coating.

Advantageously, the protective weight coating has a depth of between 60 to 80 mm.

Preferably, the anti-corrosion coating is an epoxy resin.

One embodiment of the present invention will now be described with reference to the accompanying drawings in which:
Figure 1 is a schematic cross-sectional view of a pipe coated with a coating according to one aspect of the present invention;
Figure 2 is a schematic cross-sectional view of the pipe of Figure 1 provided with a slot for carrying an induction cable; and
Figure 3 is a schematic view of an apparatus for inserting the induction cable into the slot of Figure 2.

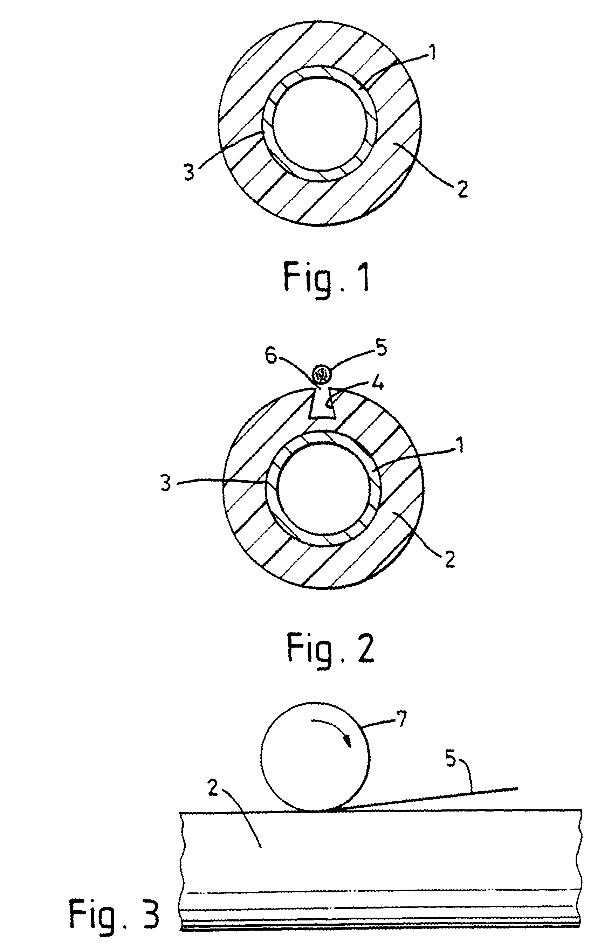

Turning to the Figures, Figure 1 shows a schematic cross-sectional view of a pipe 1 having a protective weight coating 2 applied thereto according to the present invention. The pipe is first provided with a fusion bonded anti-corrosion epoxy coating 3 of a thickness of up to about 500 µm (microns) onto which the protective weight coating is applied through a molding process.

The protective weight coating 2 comprises a compound containing a urethane plastics material and a high density filler material. The preferred filler is micro fine particulate barium sulphate which is dosed into the urethane during the molding process. The weight coating may be applied onto the pipe up to any thickness as required but is generally of the order of 60 to 80 mm. The coating is provided along substantially the whole length of the pipe, ending adjacent the ends of the pipe in order to allow successive pipes to be joined together in a process to be described further below.

A longitudinal slot 4 is cut into the coating to allow for the insertion of a heat induction cable 5 at the time of laying the pipeline. The slot is frustoconical in cross-section as shown in Figure 2, with the width of the slot increasing radially inwards from the surface of the coating. The width of the opening 6 of the slot 4 in the surface of the coating 2 is just less than the diameter of the cable 5 to be inserted as will be described below. The depth of the slot 4 is generally half the depth of the coating.

In the preferred embodiment of the invention, three such slots 4 are provided in the coating, the slots being equispaced around the surface of the coating.

The process of laying a pipeline will now be briefly described. A plurality of pre-manufacture pipe sections, each of which is provided with a coating as described above, are loaded onto a pipe-laying barge of a known type and transported to the area where the pipeline is to be laid.

The pipe sections are laid end to end on conveyors on the barge and carried to a coupling station. Referring to Figure 3 there is shown a schematic view of a roller 7 carried on the barge for laying the pipeline, the roller being adapted to move across the surface of the pipeline sections to press the induction cable 5 into the longitudinal slot 4. As shown in Figure 2, the cable 5 is oversized for the outer width 6 of the slot so that once the cable is inserted by a "snap-fit" into the slot it is securely retained therein. Due to the configuration of the slots, water can circulate around the induction cables thereby allowing preventing any localised scorching of the protective weight coating. Furthermore, the cables are easy to replace in the event of damage or failure of the cable.

During transfer of the pipe sections along the conveyors, the free ends of adjacent pipe sections may be welded together in one or more welding operations prior to the following bonding process.

The ends of two adjacent pipes are encapsulated in a jacket and a urethane is pumped into the jacket between the ends of the pipes. The urethane is a fast acting plastics material which will cure quickly and undergo a chemical reaction with the urethane in the weight and insulating coating to chemically bond the connecting section to the general coating. This gives the pipeline 100% integrity along its length.

The pipe line is then played out from the end of the barge and lowered onto the sea bed. Due to the flexibility of the weight and insulating coating described above, the pipeline can withstand the tensile stress placed on it during the laying process and the pipeline does not suffer damage during this process.

Such a weight and insulating coating 2 formed of the above materials has been found to provide in the region of five times the insulating effect of concrete.

Due to the non-carbon and non-magnetic properties of the weight and insulating coating 2 described above, heat is not drawn from the induction heating system as has previously been the case with concrete coatings where heat was lost from the induction system to heat the steel cages around the pipeline. Therefore, the induction system for the pipeline works more efficiently with the above described coating than with previously used concrete insulating coatings.

Furthermore, due to the absence of voids in the above described system, it is anticipated that zero creep will occur during the life of the pipeline due to pressure of the water surrounding the pipeline.

It is envisaged that the coating described above will be used on pipelines laid to a depth of approx 1500 m and in temperatures between 0°C and 100°C.

## Claims

1. A pipe coating with a high density filler material, characterized in that it comprises an anti-corrosion coating having a film thickness of from about 350 µm to about 500 µm, and a protective weight coating of a urethane based copolymer applied over said anti-corrosion coating, said protective weight coating incorporating the high density filler material.

2. A pipe coating according to claim 1, wherein the high density filler material is micro fine particulate barium sulphate.

3. A pipe coating according to claim 1 or 2, wherein a slot is provided in the surface of the protective weight coating for laying an induction cable.

4. A pipe coating according to claim 3, wherein the slot is frustoconical in section.

5. A pipe coating according to claim 4, wherein the width of the slot increases away from the surface of the protective weight coating.

6. A pipe coating according to any one of claims 3 to 5, wherein three slots are provided in the surface of the protective weight coating, the slots being equispaced around the circumference of the coating.

7. A pipe coating according to any one of the preceding claims, wherein the protective weight coating has a depth of between 60 to 80 mm.

8. A pipe coating according to any one of the preceding claims, wherein the anti-corrosion coating is an epoxy resin.

9. A pipe coating according to any one of the preceding claims wherein the protective weight coating is a substantially void free polyurethane.

10. A pipe suitable for subsea use coated with a pipe coating according to any one of the preceding claims.

## Patentansprüche

1. Rohrbeschichtung mit einem hochdichten Füllmaterial, dadurch gekennzeichnet, daß es eine Anti-Korrosionsbeschichtung, die eine Filmdicke von etwa 350 µm bis etwa 500 µm aufweist, und eine schützende Gewichtsbeschichtung aus einem auf Urethan basierenden Copolymer umfaßt, welche über der Anti-Korrosionsbeschichtung aufgebracht ist, wobei die schützende Gewichtsbeschichtung das hochdichte Füllmaterial einschließt.

2. Rohrbeschichtung nach Anspruch 1, wobei das hochdichte Füllmaterial mikrofeines partikuliertes Barium-Sulfat darstellt.

3. Rohrbeschichtung nach Anspruch 1 oder 2, wobei ein Schlitz in der Oberfläche der schützenden Gewichtsschicht vorgesehen ist, um ein Induktionskabel zu legen.

4. Rohrbeschichtung nach Anspruch 3, wobei der Schlitz im Querschnitt kegelstumpfförmig ist.

5. Rohrbeschichtung nach Anspruch 4, wobei die Breite des Schlitzes mit zunehmendem Abstand von der Oberfläche der schützenden Gewichtsschicht zunimmt.

6. Rohrbeschichtung nach einem der Ansprüche 3 bis 5, wobei die Schlitze in der Oberfläche der schützenden Gewichtsschicht vorgesehen sind, und die Schlitze um den Umfang der Beschichtung im gleichen Abstand angeordnet sind.

7. Rohrbeschichtung nach einem der vorhergehenden Ansprüche, wobei die schützende Gewichtsschicht eine Tiefe von 60 bis 80 mm aufweist.

8. Rohrbeschichtung nach einem der vorhergehenden Ansprüche, wobei die Anti-Korrosionsbeschichtung ein Epoxyharz darstellt.

9. Rohrbeschichtung nach einem der vorhergehenden Ansprüche, wobei die schützende Gewichtsbeschichtung im wesentlichen hohlraumfreies Polyurethan ist.

10. Rohr, geeignet für Unterwasser-Verwendung, beschichtet mit einem Rohrschutz gemäß einem der vorhergehenden Ansprüche.

## Revendications

1. Revêtement de tube ayant une matière de charge haute densité, caractérisé en ce qu'il comprend un revêtement ànticorrosion ayant une épaisseur de feuil d'environ 350 µm à environ 500 µm, et un revêtement de lestage et de protection constitué d'un copolymère à base d'uréthanne, appliqué sur ledit revêtement anticorrosion, ledit revêtement de lestage et de protection contenant la matière de charge haute densité.

2. Revêtement de tube selon la revendication 1, dans lequel la matière de charge haute densité est du sulfate de baryum particulaire, à granulométrie micronique.

3. Revêtement de tube selon la revendication 1 ou 2, dans lequel une fente est pratiquée dans la surface du revêtement de lestage et de protection pour le dépôt d'un câble d'induction.

4. Revêtement de tube selon la revendication 3, dans lequel la fente a une section tronconique.

5. Revêtement de tube selon la revendication 4, dans lequel la largeur de la fente augmente au fur et à mesure qu'on s'éloigne de la surface du revêtement de lestage et de protection.

6. Revêtement de tube selon l'une quelconque des revendications 3 à 5, dans lequel trois fentes sont pratiquées dans la surface du revêtement de lestage et de protection, les fentes étant disposées de manière équidistante autour de la circonférence du revêtement.

7. Revêtement de tube selon l'une quelconque des revendications précédentes, dans lequel le revêtement de lestage et de protection a une profondeur comprise entre 60 et 80 mm.

8. Revêtement de tube selon l'une quelconque des revendications précédentes, dans lequel le revêtement anticorrosion est une résine époxy.

9. Revêtement de tube selon l'une quelconque des revendications précédentes, dans lequel le revêtement de lestage et de protection est un polyuréthanne essentiellement exempt de vides.

10. Tube approprié pour une utilisation sous-marine avec un revêtement de tube selon l'une quelconque des revendications précédentes.
